# EUROPÄISCHE PATENTSCHRIFT

(11) **EP 0 959 987 B1**
(45) Veröffentlichungstag und Bekanntmachung des Hinweises auf die Patenterteilung: **08.08.2001**
(21) Anmeldenummer: 97954464.0
(22) Anmeldetag: 23.12.1997
(51) Int. Cl.: B01J 31/24, C07F 9/50, C07C 5/05, C07C 209/52, C07C 45/50, C08F 38/00

(54) **VERWENDUNG PERFLUORALKYLSUBSTITUIERTER PHOSPHORVERBINDUNGEN ALS LIGANDEN FÜR DIE HOMOGENE KATALYSE IN ÜBERKRITISCHEM KOHLENDIOXID**
USE OF PERFLUOROALKYL SUBSTITUTED PHOSPHORUS COMPOUNDS AS LIGANDS FOR HOMOGENEOUS CATALYSIS IN SUPERCRITICAL CARBON DIOXIDE
UTILISATION DE COMPOSES PHOSPHORES PERFLUOROALKYL-SUBSTITUES COMME LIGANDS POUR LA CATALYSE HOMOGENE DANS DU DIOXYDE DE CARBONE SUR-CRITIQUE

(30) Priorität: 23.01.1997 DE 19702025
(43) Veröffentlichungstag der Anmeldung: 01.12.1999
(73) Patentinhaber: Studiengesellschaft Kohle mbH, 45470 Mülheim (DE)
(72) Erfinder: LEITNER, Walter, D-45470 Mülheim (DE); KAINZ, Sabine, D-45470 Mülheim (DE); KOCH, Daniel, D-45470 Mülheim (DE); WITTMANN, Klaus, D-45470 Mülheim (DE); SIX, Christian, D-45470 Mülheim (DE)
(74) Vertreter: von Kreisler, Alek, Dipl.-Chem.
(86) Internationale Anmeldenummer: EP9707266
(87) Internationale Veröffentlichungsnummer: WO9832533

(56) Entgegenhaltungen:
- EP-A- 0 646 590
- EP-A- 0 652 202
- US-A- 5 198 589
- US-A- 5 567 856

## Beschreibung

Die vorliegende Erfindung betrifft die Verwendung von Phosphorverbindungen, die eine oder mehrere Perfluoralkylketten der allgemeinen Formel R¹ [R¹= -(CH₂)ₓ(CF₂)_{y}E (E = F, H, x = 0 - 4, y = 2 - 12)] enthalten, als Katalysatoren oder Bestandteile von Katalysatoren in einem Reaktionsgemisch bestehend im wesentlichen aus den Reaktanden, dem Katalysator und Kohlendioxid im überkritischen Zustand (scCO₂). Insbesondere wurde gefunden, daß die Einführung von Perfluoralkylseitenketten R¹ in die Arylreste von unlöslichen oder schlecht löslichen Arylphosphorverbindungen zu einer deutlichen Verbesserung ihrer Löslichkeit in scCO₂ führt, so daß sie überraschenderweise sogar die Löslichkeit von bekanntermaßen löslichen Alkylphosphorverbindungen übertrifft. Die Erfindung betrifft weiterhin die Verwendung derartiger Reaktionsgemische zur chemoselektiven Hydrierung von Polyenen zu Monoenen zur Hydroformylierung von Olefinen, zur enantioselektiven Hydrierung von Iminen und für C-C-Verknüpfungsreaktionen.

Kohlendioxid im überkritischen Zustand (scCO₂) ist ein interessantes Lösungsmittel für die Durchführung katalytischer Reaktionen, da es im Gegensatz zu konventionellen organischen Lösungsmitteln toxikologisch und ökologisch unbedenklich ist. Weiterhin besitzt scCO₂ die Eigenschaft, mit vielen gasförmigen Reaktionspartnern in weiten Grenzen vollständig mischbar zu sein, wodurch die bei Gas/Flüssigphasen-Reaktionen nicht selten auftretende Limitierung der Reaktionsgeschwindigkeit durch Diffusionsprozesse grundsätzlich vermieden wird. Aufgrund der mit Druck und Temperatur variablen Lösungseigenschaften von scCO₂ ist ferner bei geeigneter Wahl der äußeren Parameter in günstigen Fällen die Abtrennung von Haupt- oder Nebenprodukten aus der Reaktionsmischung möglich. Ein Überblick über katalytische Reaktionen in scCO₂ findet sich in *Science* **1995,** *269*, 1065.

Die Verwendung von Phosphorverbindungen als Katalysatoren oder Bestandteile von Katalysatoren in scCO₂ wird in EP 0 652 202 A1 für die Synthese von Ameisensäure und ihrer Derivate beschrieben. Eine in *J*. *Am*. *Chem*. *Soc.* **1996,** *118*, 344 veröffentlichte Studie zeigt jedoch, daß nur Phosphorverbindungen mit Alkylresten effektive Katalysatoren für diesen Prozeß bilden, während Arylphosphorverbindungen aufgrund ihrer schlechten Löslichkeit in scCO₂ nicht genutzt werden können. Allerdings zeichnen sich Arylphosphorverbindungen gegenüber Alkylphosphorverbindungen durch eine deutlich geringere Empfindlichkeit gegenüber Sauerstoff aus und sind synthetisch in der Regel erheblich leichter zugänglich, so daß ihr Einsatz eine deutliche Vereinfachung der technischen Durchführung von katalytischen Verfahren bedeutet. Arylverbindungen stellen auch die bei weitem überwiegende Mehrzahl chiraler Phosphorverbindungen dar, die als Katalysatoren oder Bestandteile von Katalysatoren in der asymmetrischen Katalyse verwendet werden (H. Brunner, W. Zettelmeier, *Handbook of Enantioselective Catalysis,* VCH, Weinheim, **1993).** Die Löslichkeit von Arylphosphorverbindungen in scCO₂ kann durch partielle oder vollständige Hydrierung der Arylreste zu gesättigten Alkylgruppen erhöht werden (*Tetrahedron Letters* **1996,** *37,* 2813). Der Effekt ist jedoch gering und zudem mit den bereits erwähnten Nachteilen von Alkylphosphorverbindungen gekoppelt. Ferner ist die Methode mit einem hohen synthetischen Aufwand verbunden und nicht allgemein auf Arylphosphorverbindungen übertragbar. In der gleichen Arbeit wird auch die Zugabe von Perfluoralkylalkoholen zur Reaktionsmischung als Möglichkeit zur Erhöhung der Löslichkeit vorgeschlagen. Auch diese Methode führt nur zu einer geringen Verbesserung der Löslichkeit und erschwert zusätzlich aufgrund des unerwünschten Einsatzes größerer Mengen der Additive eine mögliche Nutzung der oben genannten Vorteile von scCO₂.

Der vorliegenden Erfindung lag also das Problem zugrunde, eine allgemeine Methode zur Erhöhung der Löslichkeit von Arylphosphorverbindungen in scCO₂ zu finden, um sie als Katalysatoren oder Bestandteile von Katalysatoren in Reaktionsgemischen bestehend im wesentlichen aus den Reaktanden, dem Katalysator und scCO₂ nutzen zu können.

Es wurde gefunden, daß die Anbindung einer oder mehrerer Perfluoralkylgruppen R¹ an Phosphorverbindungen eine generelle Methode ist, um ihre Löslichkeit in scCO₂ zu erhöhen und sie als Katalysatoren oder Bestandteile von Katalysatoren in Reaktionsgemischen bestehend im wesentlichen aus den Reaktanden, dem Katalysator und scCO₂ nutzen zu können. R¹ ist dabei ein Rest der allgemeinen Formel -(CH₂)ₓ(CF₂)_{y}E (E = F, H, x = 0 - 4, y = 2- 12), bevorzugt sind Reste R¹ mit E = F, x=2 und y=2-10, besonders bevorzugt y=6-8.

Insbesondere wurde gefunden, daß die Einführung von Perfluoralkylseitenketten R¹ in die Arylreste von unlöslichen oder schlecht löslichen Arylphosphorverbindungen zu einer deutlichen Verbesserung ihrer Löslichkeit in scCO₂ führt, so daß sie überraschenderweise sogar die Löslichkeit von bekanntermaßen löslichen Alkylphosphorverbindungen erheblich übertrifft.

Außerdem wurde gefunden, daß die mit R¹ substituierten Phosphorverbindungen als Bestandteile von Katalysatoren für die chemoselektive Hydrierung von Polyenen zu Monoenen, für die Hydroformylierung von Olefinen in scCO₂, zur enantioselektiven Hydrierung von Iminen und für C-C-Verknüpfungsreaktionen eingesetzt werden können.

### Synthese und Eigenschaften von perfluoralkylsubstituierten Phosphorverbindungen:

Die Synthese von Phosphorverbindungen, die mindestens eine Perfluoralkylkette der allgemeinen Formel R¹ enthalten, wird in der Anmeldung EP 0 633 062 A1 beschrieben. Arylphosphorverbindungen mit fluorierten Alkylketten sind auch in

EP 0 646 590 A1 beschrieben. In der vorliegenden Erfindung wurde für die Synthese perfluoralkylsubstituierter Arylphosphorverbindungen die in Schema 1 zusammengefaßte Syntheseroute gewählt.

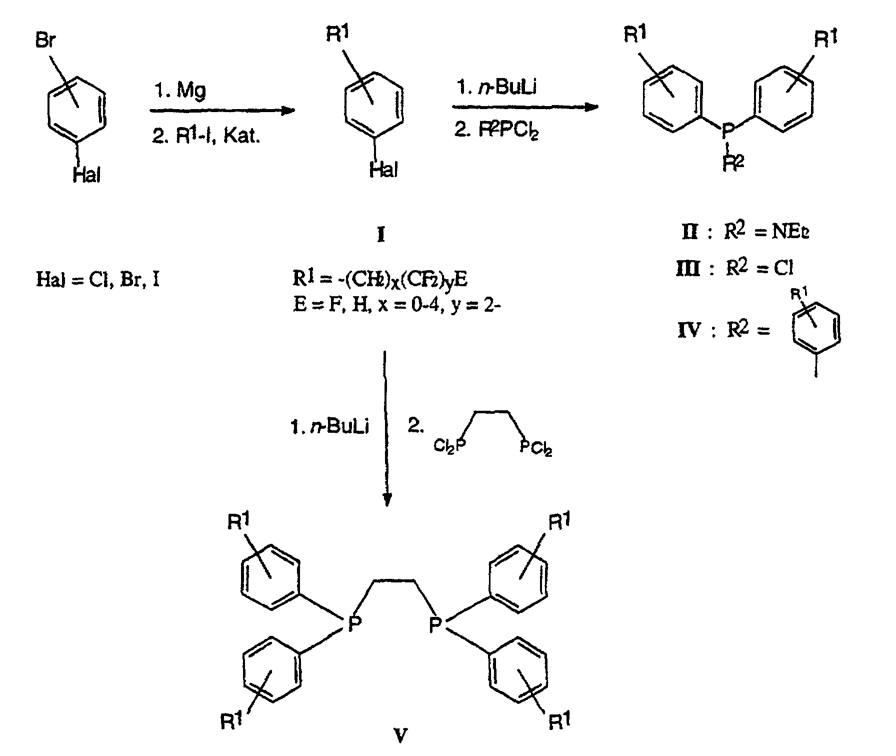

### Schema 1: Syntheseroute für perfluoralkylsubstituierte Arylphosphorverbindungen.

Sowohl die Anzahl und Position der Substitution als auch die Länge der -(CH₂)ₓ-Kette und der perfluorierten Kette -(CF2)y⁻ sind in dieser Synthese variabel. Für die bevorzugte Form von R¹ (x=2, y=6-8) wurden die Kupplungsprodukte **I** aus den nach üblichen Methoden erzeugten Grignard-Verbindungen und den kommerziell erhältlichen Iodiden R¹-I in Gegenwart eines Übergangsmetallkatalysators, bevorzugt einer Kupferverbindung, erhalten (Tabelle 1).

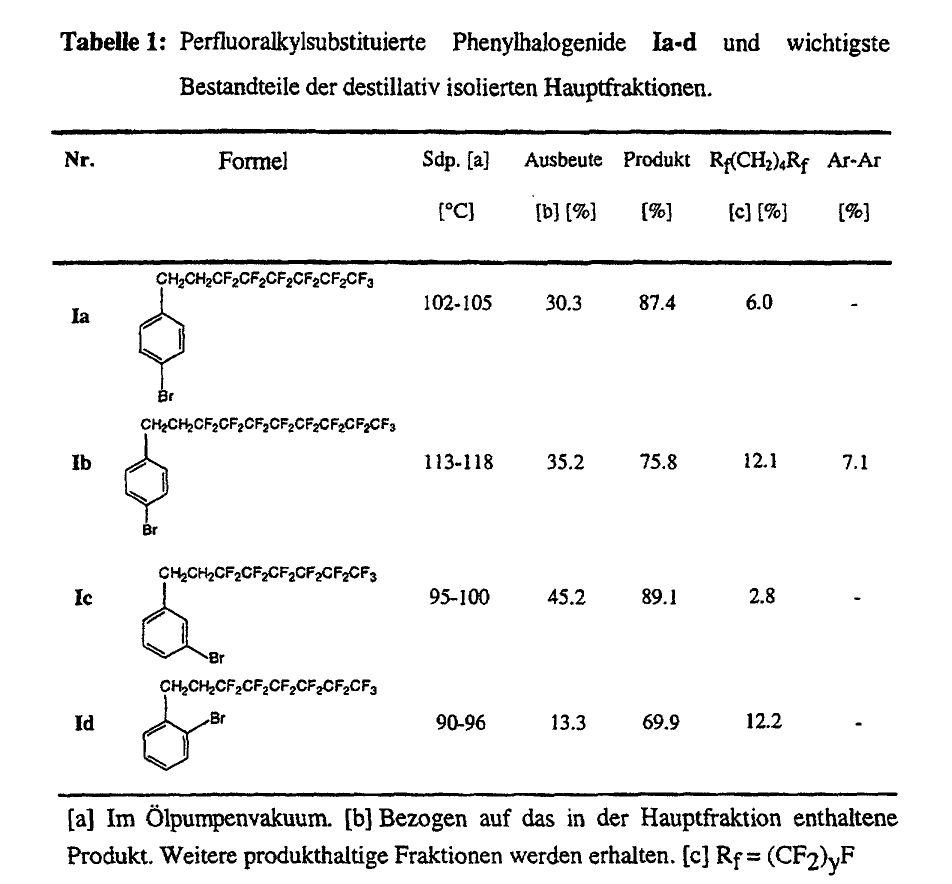

Metallierung der Arylhalogenide, bevorzugt mit *n*-BuLi, und anschließende Kupplung mit Phosphorverbindungen R²_{z}PR³_{3-z} (R² = beliebiger Alkyl- oder Arylrest oder = R³, R³ = reaktive Gruppe, z. B. Halogen, NR²₂, OR², z = 0-2) führt zu den Arylphosphorverbindungen **II-V** (Tabelle 2). Ausgehend von Verbindungen **II** und **III** lassen sich weitere Phosphorverbindungen synthetisieren, wie Schema 2 verdeutlicht. Formel **VII** repräsentiert beispielsweise die Verbindungsklasse der Phosphinite, die Brücke -X- kann in **VI** und **VII** chiral oder achiral sein.

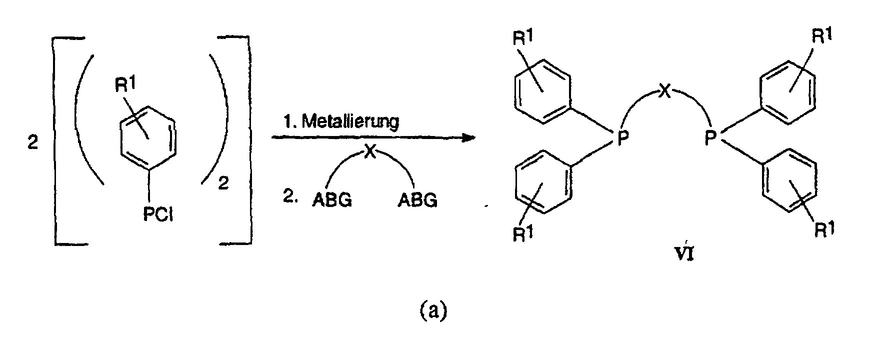

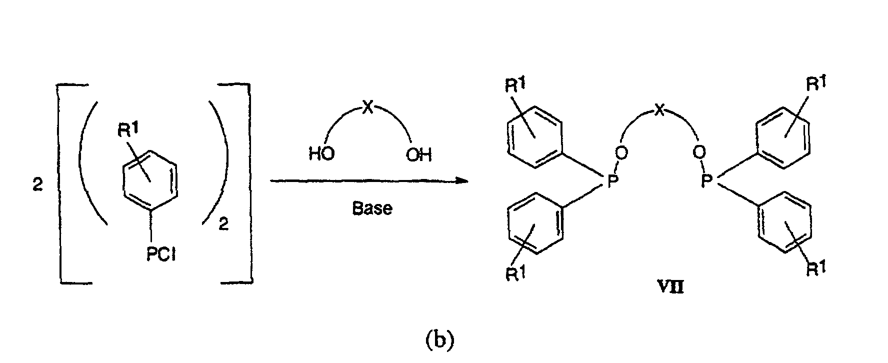

### Schema 2: Weitere Methoden zur Synthese chiraler und achiraler Arylphosphorverbindungen mit Perfluoralkylseitenketten ausgehend von Verbindungen vom Typ III. (a) Synthese von zweizähnigen Phosphanen VI, (b) Synthese von zweizähnigen Phosphiniten VII. - (ABG = Abgangsgruppe, z. B. Br, Tosylat.; -X-= chirales oder achirales Kohlenstoffgerüst)

Die perfluoralkylsubstituierten Arylphosphorverbindungen können zur Synthese von Metallkomplexen nach konventionellen Methoden verwendet werden. Repräsentative Beispiele **VIII-X** sind in Tabelle 3 zusammengefaßt, ohne daß durch die Wahl der Metalle oder der Komplextypen eine Beschränkung impliziert wird.

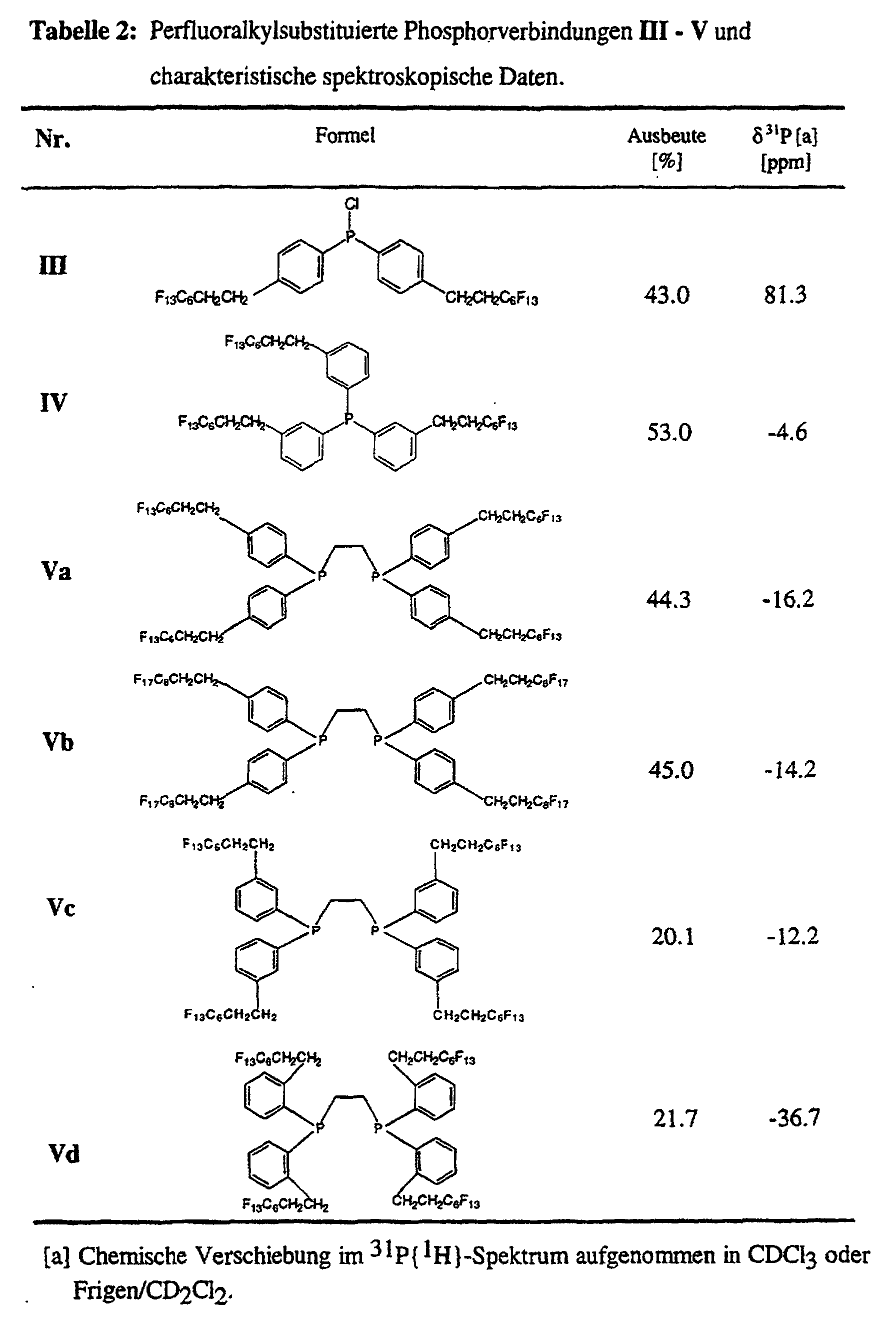

**Tabelle 3:**

| Metallkomplexe **VIII-X** mit perfluoralkylsubstituierten Phosphorverbindungen als Liganden und charakteristische spektroskopische Daten [a]. | | | | | |
|---|---|---|---|---|---|
| **Nr.** | Formel | δ(¹³Cᵢₚₛₒ) [b] [ppm] | δ(³¹P) [ppm] | *J* [Hz] | δ(¹⁰³Rh) [ppm] |
| **VIII** | [**(IV)**₃RhCl] | nicht bestimmt | 51.1 (dt) 36.2 (dd) | 188, 38 142, 38 | nicht bestimmt |
| **IXa** | [**(Va)**Rh(hfacac)] | 134.7 (m) | 70.5 (d) | 196 | 459 |
| **IXb** | [**(Vb)**Rh(hfacac)] | 134.7 (m) | 70.5 (d) | 196 | 459 |
| **IXc** | [**(Vc)**Rh(hfacac)] | 137.0 (m) | 71.8 (d) | 196 | 461 |
| **IXd** | [**(Vd)**Rh(hfacac)] | 134.0 (m) | 66.5 (d) | 197 | 705 |
| **X** | *trans-* [**(Va)**₂RuCl₂] | nicht bestimmt | 43.9 | - | - |
| [a] in THF-d₈ oder CDCl₃. [b] Cᵢₚₛₒ bezeichnet das direkt am P-Atom gebundene C-Atom des Arylrests. | | | | | |

### Erhöhung der Löslichkeit in scCO₂

Die Einführung der Perfluoralkylgruppen R¹ in Phosphorverbindungen erhöht deren Löslichkeit in scCO₂ in einem Maße, daß diese als effektive Katalysatoren in scCO₂ verwendet werden können; dies gilt auch für Alkylphosphorverbindungen, insbesondere jedoch für Arylphosphorverbindungen. Am Beispiel der Komplexe [{R⁴P(CH₂)₂PR⁴}Rh(hafac)] **IX** (hfacac = Hexafluoroacetylacetonat) wird dieser Effekt im folgenden quantitativ beschrieben.

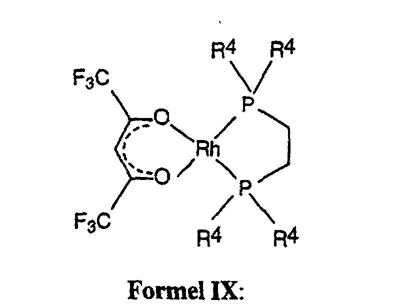

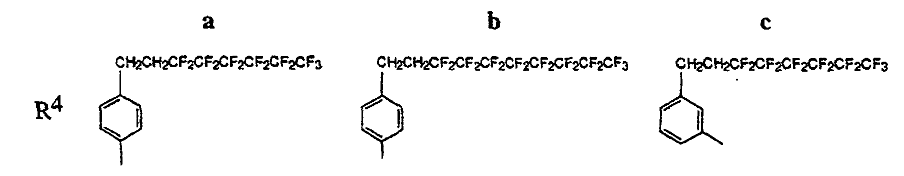

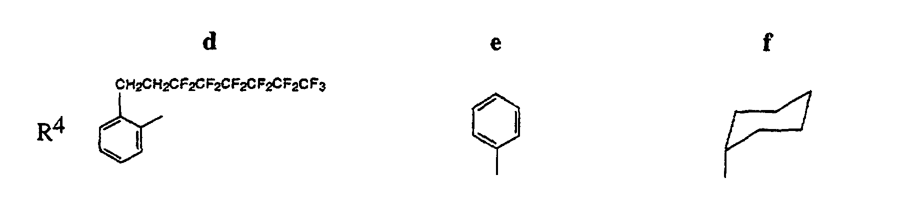

Die Löslichkeit der Komplexe **IXa,b** in scCO₂ wurde mittels UV-Spektroskopie bestimmt und mit der Löslichkeit der unsubstituierten Stammverbindung [(dppe)Rh(hfacac)] **IXe** verglichen. **IXe** ist selbst bei hoher Dichte ρ und damit hoher Lösungskraft des scCO₂ (*Angew*. *Chem.* **1978,** *90, 748) nicht* löslich (*T* = 50 °C, *ρ* = 110 bar, p = 0.75 g cm⁻³). Mit **IXa** und **IXb** werden hingegen bereits bei niedrigen Dichten (*T*= 50 °C, *ρ* = 90 bar, ρ = 0.55 g cm⁻³) gelb-orange Lösungen erhalten. Aus den Absorptionsmaxima der UV-Spektren (siehe Abb. 1) läßt sich über das Lambert-Beer'sche-Gesetz mittels Vergleichsdaten für **THF**-Lösungen eine Sättigungskonzentration von 6.3 x 10⁻⁵ M für **IXa** und 7.5 x 10⁻⁵ M für **IXb** ermitteln. Zum Vergleich ist in Abb. 1 auch das UV-Spektrum des Komplexes **IXf** mit der Alkylphosphorverbindung 1,2-Bis(dicyclohexylphosphino)ethan (dcpe) aufgeführt. Die Löslichkeit von **IXf** in scCO₂ beträgt unter identischen Bedingungen 1.1 x 10⁻⁵ M, ist also um 5x bzw. 7x geringer als die von **IXa** und **IXb.**

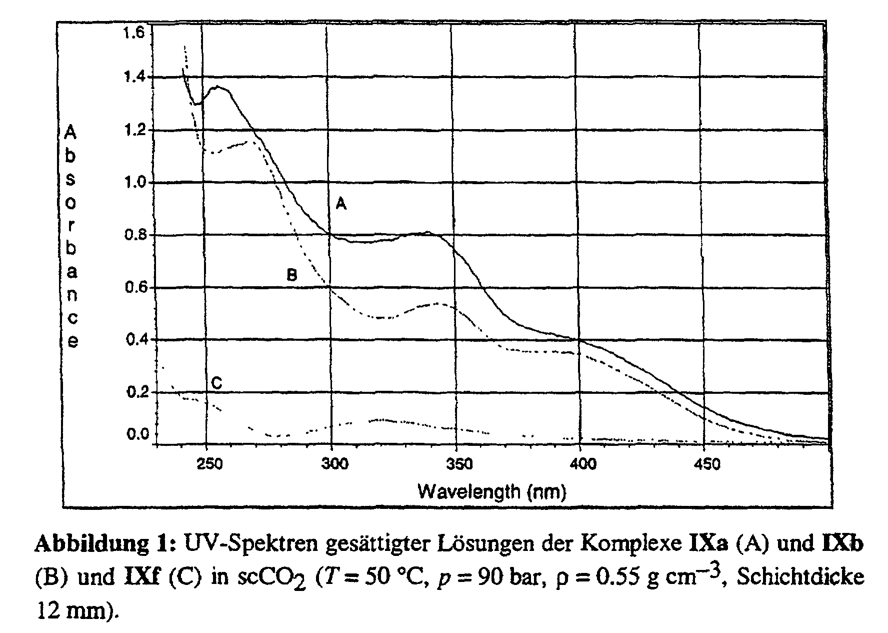

Mit zunehmender Dichte der überkritischen Phase steigt die Löslichkeit von perfluoralkylsubstituierten Phosphorverbindungen weiter an. Bei einer Dichte von ρ = 0.75 g cm⁻³ (*T*= 50 °C, ρ = 110 bar) lösten sich beispielsweise 158 mg **IXa** in einer Hochdruckzelle (*V* = 17.3 cm⁻³) vollständig auf, so daß als Untergrenze der Löslichkeit unter diesen Bedingungen ein Wert von 4.4 x 10⁻³ M abgeschätzt werden kann. Die Löslichkeit von **IXa** ist also *mindestens* 150mal höher als die von Burk et al. in *J. Am*. *Chem*. *Soc.* **1995,** *117*, 8277 für den Alkylphosphorkomplex [(Duphos)Rh(η⁴-C₈H₁₂)]⁺ gefundene maximale Löslichkeit von 0.03 x 10⁻³ **M** (*T* = 50 °C, *p = 345* bar).

### Verwendung von perfluoralkylsubstituierten Phosphorverbindungen als Katalysatoren bzw. Bestandteile von Katalysatoren in scCO₂.

Die Einführung von Perfluoralkylseitenkette der allgemeinen Formel **I** macht somit aus der unlöslichen Stammverbindung [(dppe)Rh(hfacac)) **IXe** nicht nur eine perfluoralkylsubstituierte Arylphosphorverbindung **IXa,** die in scCO₂ löslich ist, sondern überraschenderweise sogar eine Löslichkeit aufweist, die deutlich über der von bekannten löslichen Alkylphosphorverbindungen liegt. Damit werden insbesondere Arylphosphorverbindungen, die sich durch eine im allgemeinen höhere Stabilität gegenüber Sauerstoff und damit eine leichtere Handhabung als Alkylphosphorverbindungen auszeichnen, zur Verwendung als Katalysatoren oder Bestandteile von Katalysatoren in scCO₂ verfügbar. Die Verbindungen **IV** sind beispielsweise perfluoralkylsubstituierte, in scCO₂ lösliche Analoga des in organischen Lösungsmitteln vielfältig als Katalysator oder Bestandteil von Katalysatoren eingesetzten Triphenylphosphins (TPP). Die Verbindungen **V** sind Analoga des ebenfalls häufig verwendeten zweizähnigen Liganden 1,2-Bis(diphenylphosphino)ethans (dppe).

In scCO₂ lösliche Katalysatoren, die aus perfluoralkylsubstituierten Arylphosphorverbindungen und einem Übergangsmetall bestehen, können *in situ* aus geeigneten metallhaltigen Vorläuferkomplexen und den Phosphorverbindungen gebildet werden, oder es können vorgefertigte Komplexe wie **VIII-X** verwendet werden. Beispiele für mögliche Übergangsmetallkomponenten sind Ni, Pd, Pt, Co, Rh, Ir, Fe, Ru, Os, Ti, W, Mo und Verbindungen dieser Metalle. Beispiele für katalytische Prozesse sind Hydrierungen, Oxidationen, C-C-Verknüpfungen und Polymerisationen. Die im folgenden beschriebenen Beispiele demonstrieren das Potential dieser Katalysatoren in scCO₂, ohne daß dadurch eine Beschränkung möglicher Anwendungen impliziert werden soll. Insbesondere wird in dem Beispiel der Hydroformylierung der entscheidende positive Einfluß der Perfluoralkylkette R¹ belegt.

### a) Chemoselektive Hydrierung von Polyenen zu Monoenen

Die chemoselektive Hydrierung von Polyenen zu Monoenen in scCO₂ in Gegenwart von Katalysatoren auf Basis von perfluoralkylsubstituierten Arylhosphorverbindungen wird zweckmäßigerweise so durchgeführt, daß eine Mischung aus Substrat, H₂ und komprimiertem CO₂ in einem Hochdruckreaktor auf eine Temperatur oberhalb des kritischen Punkts der Mischung aufgeheizt wird. Die chemoselektive Hydrierung von Polyenen in scCO₂ in Gegenwart von perfluoralkylsubstituierten Phosphorverbindungen kann bei Temperaturen ab 30°C, bevorzugt zwischen 40°C und 100°C und bei Partialdrücken von H₂ zwischen 1 und 100 bar, bevorzugt zwischen 1 und 50 bar durchgeführt werden. Der Gesamtdruck liegt bei 100 bis 300 bar, bevorzugt zwischen 180 und 250 bar. In diese Mischung wird der Katalysator, beispielsweise **IXa**/NEt₃, eingebracht. Das Fortschreiten der Reaktion kann anhand der Druckabnahme aufgrund des H₂-Verbrauchs verfolgt werden. Eine aufwendige und lösungsmittelintensive Aufarbeitung der Reaktionsmischung kann beim Arbeiten in scCO₂ entfallen, die Kohlenwasserstoffe werden beispielsweise durch einfaches Einkondensieren in einer Kühlfalle praktisch rein gesammelt. Am Beispiel der Hydrierung von Isopren wurde gezeigt, daß mit **IXa** in scCO₂ vergleichbare Selektivitäten für die Bildung der einfach ungesättigten Produkte erzielt werden, wie mit dem verwandten Katalysator [{Ph₂P(CH₂)₃PPh₂}Rh(η³-C₈H₁₁)] in organischen Lösungsmitteln (Tabelle 4). Der Anteil an 3-Methylbuten-1 ist in scCO₂ sogar etwas höher als in konventionellen Lösungsmitteln.

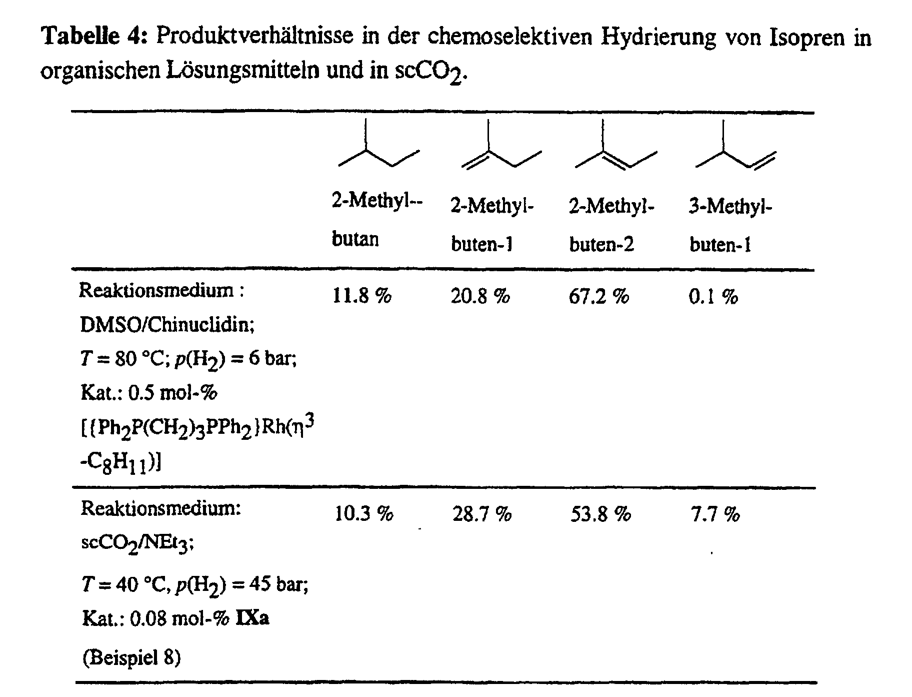

### b) Hydroformylierung von 1-Octen

Die Cobalt-katalysierte Hydroformylierung in scCO₂ ohne Phosphanzusatz ist in US 5 198 589 beschrieben. Rhodiumkomplexe mit Phosphorliganden bieten in gängigen Lösungsmitteln jedoch viele Vorteile gegenüber Cobalt-Katalysatoren in der Hydroformylierung und werden daher in der Technik vermehrt genutzt (B. Cornils, W. A. Herrmann (Hrsg.), *Applied Homogeneous Catalysis with Organometallic Compounds*, Vol. 1, VCH, **1996,** S. 7f). Die Übertragung des in organischen Lösungsmitteln technisch etablierten Katalysatorsystems Rh/TPP auf die Anwendung in scCO₂ scheitert jedoch an der Unlöslichkeit der aktiven Spezies in diesem Medium. Bis zum Erreichen des überkritischen Zustands verläuft die Reaktion in geringem Umfang aufgrund der Löslichkeit des Katalysators im Olefin. Am kritischen Punkt wird der Katalysator unlöslich, die überkritische Phase aus Olefin und CO₂ ist dann farblos und es erfolgt kein weiterer Umsatz des Olefins.

Mit dem perfluoralkylsubstituierten TPP-Analogon **IV** verläuft die rhodiumkatalysierte Hydroformylierung von Olefinen hingegen in scCO₂ glatt (Beispiel 10), wobei die Löslichkeit der katalytisch aktiven Spezies unter diesen Bedingungen durch eine intensive Gelbfärbung der überkritischen Phase angezeigt wird. Unter überkritischen Bedingungen werden in der Hydroformylierung von 1-Octen mit einem Rh/**IV**-Katalysator beispielsweise 92 % Umsatz zu 1-Nonanal und 2-Nonanal und ein n/iso-Verhältnis von 4.6 erzielt. Die entsprechenden Werte des Kontrollexperiments mit unsubstituiertem TPP (Beispiel 9) betragen 26 % und 3.5. Dieses Beispiel belegt die Bedeutung der Perfluoralkylketten für die Nutzung der Arylphosphane als Katalysatoren oder Bestandteile von Katalysatoren in scCO₂. Die Hydroformylierung von Olefinen in scCO₂ in Gegenwart von Katalysatoren, die perfluoralkylsubstituierte Phosphorverbindungen enthalten, wird zweckmäßig so durchgeführt, daß eine Mischung aus Katalysator, Olefin, CO, H₂ und CO₂ in einem Hochdruckreaktor auf eine Temperatur oberhalb des kritischen Punkts der Mischung aufgeheizt wird. Die rhodiumkatalysierte Hydroformylierung von Olefinen in Gegenwart von perfluoralkylsubstiuierten Phosphorverbindungen kann bei Temperaturen ab 30 °C, bevorzugt zwischen 40°C und 100°C und bei Partialdrücken von CO und H₂ zwischen 1 und 120 bar, bevorzugt zwischen 1 und 60 bar durchgeführt werden. Der Gesamtdruck liegt bei 100 bis 300 bar, bevorzugt zwischen 180 und 250 bar.

### Beispiel 1:

### Darstellung von 1-Brom-4-(1H,1H,2H,2H-perfluoroctyl)benzol (Ia):

In einem 500 ml-Dreihalskolben mit Rückflußkühler, Blasenzäbler und Tropftrichter wurden 4.30 g (0.18 mol) Mg-Späne in 15 ml Et₂O vorgelegt und anschließend 40.5 g (0.17 mol) 1,4-Dibrombenzol in 75 ml Et₂O zugetropft. Die Grignardlösung wurde 3 d bei Raumtemperatur gerührt, das überschüssige Mg abfiltriert und der Gehalt der Grignardlösung mittels Titration bestimmt (1.85 M, 94.9 %).

Zu einer Lösung von 43.8 g (92.5 mmol) lH,lH,2H,2H-Perfluoroctyliodid und 2 Löffelspatel [(η⁴-C₈H₁₂)CuCl] in 20 ml THF wurden 47.5 ml (87.9 mmol) der zuvor hergestellten Grignardlösung bei 5 °C zugetropft. Die Farbe der Reaktionsmischung veränderte sich von gelb nach braun und es entstand ein Niederschlag. Es wurde mit 100 ml THF und 100 ml Et₂O verdünnt und 4 h am Rückfluß erhitzt. Die Reaktionslösung wurde nach dem Abkühlen mit 300 ml ges. NH₄Cl-Lösung hydrolysiert. Die blaue, wäßrige Phase wurde mit 3x 50 ml Et₂O ausgeschüttelt und die vereinigten organischen Phasen mit 2x 40 ml H₂O gewaschen und über Na₂SO₄ getrocknet. Nach Entfernen des Lösungsmittels wurde ein hellbraunes dickflüssiges Rohprodukt erhalten, das im Ölpumpenvakuum über eine Vigreux-Kolonne (Länge: 19cm, Durchmesser: 16mm) fraktioniert destilliert wurde. Bei einer Siedetemperatur von 102-105 °C gingen 15.3 g eines farblosen Öls über, das nach GC-Analyse zu 87.4 % **Ia** enthielt. Eine analysenreine Probe konnte mittels präperativer GC erhalten werden.

| | | | | | |
|---|---|---|---|---|---|
| **GC-Massen- spektrum (EI)** | m/z (%) 502 (55) [M^{•+}], 423 (15) [M^{•+}-(⁷⁹Br)], 169 (100) [M^{•+}-CH₂C₆F₁₃] | | | | |
| **Elementar- analyse** | ber. gef. | C: 33.42 C: 33.55 | H: 1.60 H: 1.70 | Br: 15.88 Br: 15.97 | F: 49.09 F: 48.69 |

In analoger Weise wurden die in Tabelle 1 aufgelisteten Verbindungen **Ia-d** hergestellt.

### Beispiel 2:

### Darstellung von Di-[4-(1H,1H,2H,2H-perfluoroctyl)phenyl]chlorphosphan (III)

8.77 g (17.4 mmol) **Ia** wurden in 50 ml Et₂O gelöst und zu dieser Lösung wurden bei -50°C unter Argonatmosphäre langsam 10 ml *n*-BuLi (1.7 M in Hexan) tropfenweise zugegeben. Die trübe Lösung färbte sich hierbei intensiv gelb. Anschließend wurde die auf 0 °C erwärmte Lösung zu einer Lösung aus 1.51 g Cl₂PNEt₂ (8.6 mmol) in 20 ml THF getropft. Es wurde über Nacht im Eisbad bei 0°C gerührt, wobei sich ein Niederschlag bildete. Nach Filtration wurde die klare Reaktionslösung unter Rühren bei Raumtemperatur für ca. 30 Minuten mit HCI(g) begast. Bereits nach wenigen Minuten bildete sich ein farbloser Niederschlag, der sich im Laufe der Reaktion wieder löste. Zur Entgasung der Lösung wurde im Stickstoffbad ausgefroren, mittels Ölpumpe evakuiert und unter Argonatmosphäre wieder aufgetaut. Hierbei fiel erneut ein Niederschlag an, welcher abfiltriert wurde. Das Filtrat wurde im Vakuum auf etwa die Hälfte des ursprünglichen Volumens eingeengt und zur Kristallisation über Nacht bei -20 °C gehalten. Filtration, zweimaliges Waschen mit Et₂O und Trocknen im Hochvakuum lieferte 3.38 g **III** als farblose Kristalle
**Massenspektrum (EI)** m/z (%) 912 (100) [M^{•+}]

### Beispiel 3:

### Darstellung von Tri-[3-(1H,1H,2H,2H-perfluoroctyl)phenyl]phosphan (IV)

Zu 3.85 g (6.88 mmol) **Ic** in 10 ml Et₂O wurden unter Argonatmosphäre innerhalb von 35 Minuten 4.1 ml *n*-BuLi (1.7M in Hexan) in 10 ml Et₂O bei -25 °C getropft. Die erhaltene gelbe Lösung wurde kurzzeitig auf 0 °C erwärmt und dann wurden 284 mg (2.06 mmol) PCl₃ in 10 ml Et₂O innerhalb von 20 min. bei -10 °C zugetropft, wobei ein Niederschlag auftrat. Nach 10 min. Rühren bei Raumtemperatur wurde die Reaktionsmischung mit 20ml ges. NH₄Cl-Lösung hydrolysiert. Die wäßrige Phase wurde mit 3x 20 ml Et₂O extrahiert und die vereinigten organischen Phasen zunächst mit 2x 10 ml H₂O gewaschen und dann über Na₂SO₄ getrocknet. Abziehen des Lösungsmittels lieferte einen hellbraunen Feststoff, dessen Umkristallisation aus 10 ml eines THF/MeOH-Gemisches 1.50 g eines farblosen Feststoffs ergab, der nach GC-Analyse zu 94.4 % **IV** enthielt.
**Massenspektrum (EI):** m/z (%) 1300 (100) [M^{•+}]

### Beispiel 4:

### Darstellung von 1,2-Bis-[di-{4-(1H,1H,2H,2H-perfluoroctyl)phenyl}-phosphino]ethan (Va)

Zu 1.61 g (2.80 mmol) **Ia** in 10 ml Et₂O wurden unter Argonatmosphäre innerhalb von 10 min. 1.75 ml *n*-BuLi (1.7 M in Hexan) in 5 ml Et₂O bei -35°C getropft. Die erhaltene gelbe Lösung wurde kurzzeitig auf 0°C erwärmt und dann wurden 0.15 g (0.67 mmol) 1,2-Bis(dichlorophosphino)ethan in 7 ml Et₂O innerhalb von 25 min. bei -23°C zugetropft. Nach 10 min. Rühren bei Raumtemperatur wurde die Reaktionsmischung mit 10 ml ges. NH₄Cl-Lösung hydrolysiert, die wäßrige Phase wurde mit 2x 10 ml Et₂O extrahiert und die vereinigten organischen Phasen über Na₂SO₄ getrocknet. Das Lösungsmittel wurde entfernt und Umkristallisation des Rohprodukts aus 2-3 ml THF/MeOH lieferte 0.56 g eines farblosen Feststoffs, der nach GC-Analyse zu 93.1 % **Va** enthält.

| | | | | | |
|---|---|---|---|---|---|
| **Massen- spektrum (EI)** | m/z (%) 1782 (100) [M^{•+}], 1754 (15) [M^{•+}-CH₂CH₂], 1327 (30) [M^{•+}-(P-C₆H₄-CH₂CH₂C₆F₁₃+H)], 1313(10) [M^{•+}-(P-C₆H₄-CH₂CH₂C₆F₁₃+CH₃)], 1300(25) [M^{•+}-(P-C₆H₄-CH₂CH₂C₆F₁₃+ C₂H₄)], 877 (35) [M^{•+}-(-CH₂CH₂-P-(C₆H₄-CH₂CH₂C₆F₁₃)₂)] | | | | |
| **Elementar analyse** | ber. gef. | C: 39.08 C: 39.04 | H: 2.04 H: 2.02 | F: 55.41 F: 55.28 | P: 3.48 P: 3.47 |

In analoger Weise wurden die in Tabelle 1 aufgelisteten Verbindungen **Va-d** hergestellt.

### Beispiel 5:

### Darstellung von [(IV)₃RhCl] (VIII)

Eine Lösung von 520 mg (0.40 mmol) **IV** in 3 ml THF wurde innerhalb von 10 Minuten unter Argonatmosphäre zu einer Lösung von 17mg (0.02 mmol) [{(η²-C₈H₁₄)₂Rh(µ-Cl}₂] in 3 ml THF getropft, wobei sich die zuvor orangegelbe Lösung dunkelrot färbte. Es wurde 30 min. bei Raumtemperatur gerührt und nach Entfernen des Lösungsmittels verblieb ein dunkelroter Feststoff der aus 2 ml eines THF/MeOH-Gemisches umkristallisiert wurde. Nach Trocknen der Kristalle im Hochvakuum wurden 156.0 mg **VIII** als dunkelroter Feststoff erhalten.

### Beispiel 6:

### Darstellung von [(Va)Rh(hfacac)] (IXa):

Zu einer Lösung von 84.5 mg (0.20 mmol) [(η⁴-C₈H₁₂)Rh(hfacac)] in 10 ml THF wurden unter Argonatmosphäre 387 mg (0.20 mmol) **Va** in 10 ml THF bei -78 °C getropft. Nach Beendigung der Zugabe wurde die Reaktionslösung langsam auf Raumtemperatur erwärmt und nach ca. 20-minütigem Rühren bei Raumtemperatur wurde eine tief rotbraun gefärbte Lösung erhalten. Das Lösungsmittel wurde im Ölpumpenvakuum entfernt und der verbleibende rotbraune Feststoff (0.52g) wurde aus 5 ml eines MeOH/THF-Gemisches umkristallisiert. Nach 3 d Trocknen im Hochvakuum unter leichtem Erwärmen wurden 0.31 g des reinen Komplexes **IXa** erhalten.

| | | | | | |
|---|---|---|---|---|---|
| **Massenspektrum (EI) (Direkteinlaß)** | m/z (%) 2092 (10) [M^{•+}] | | | | |
| **Elementaranalyse** | ber. gef. | C: 36.16 C: 36.03 | H: 1.78 H: 1.85 | F: 52.65 F: 52.53 | P: 2.96 P: 2.95 |

In analoger Weise wurden die in Tabelle 1 aufgelisteten Verbindungen **IXa-d** hergestellt.

### Beispiel 7:

### Darstellung von trans-[(Va)₂RuCl₂] (X):

Eine Suspension von 0.99 g **Va** (0.55 mmol) in 120 ml Ethanol wurde unter Argonatmosphäre mit einer Lösung von 68.2 mg RuCl₃-Hydrat (35-40 % Ru, 0.23 mmol) in 4 ml entgastem Wasser versetzt. Anschließend wurde 2 h unter Rückfluß gekocht, wobei sich die zuvor grüne Suspension hellgelb färbte. Nach Filtration, Entfernen des Lösungsmittels und Trocknen im Hochvakuum wurden 0.74 g **X** erhalten.

### Beispiel 8:

### Chemoselektive Hydrierung von Isopren in scCO₂ mit IXa als Katalysator:

5.94 g Isopren (87 mmol) wurden in einen mit H₂ gespülten Druckreaktor (*V* = 100 ml) gefüllt und mit Wasserstoff wurde bei Raumtemperatur ein Druck von 45 bar (ca. 200 mmol) eingestellt. Dann wurden mittels eines Kompressors 67 g CO₂ in den Autoklaven gefüllt wobei sich im Reaktor ein Gesamtdruck von 135 bar einstellte. Anschließend wurde auf +40 °C aufgeheizt, wobei durch visuelle Kontrolle (Sichtfenster) der Übergang der Mischung in den überkritischen Bereich sichergestellt wurde. Eine Lösung von 190 mg des Rh-Komplexes **IXa** (0.09 mmol) in 2.3 g NEt₃ wurden dann mittels eines Probenaufgabesystems in den Druckreaktor eingebracht, wobei sich die zuvor farblose überkritische Phase orange-gelb färbte. Nach 19 h wurde entspannt und die Kohlenwassertstoffe in einer mit Aceton/Trockeneis gekühlten Kühlfalle einkondensiert. Die Produktverteilung wurde mittels GC ermittelt.

### Beispiel 9:

### Hydroformylierung von 1-Octen in scCO₂ mit [(η⁴-C₈H₁₂)Rh(hfacac)]/IV als Katalysator:

17.4 mg [(η⁴-C₈H₁₂)Rh(hfacac)] (0.05 mmol) und 352 mg **IV** (0.27 mmol) wurden in einem mit Argon gespülten Druckreaktor (*V* = 20 ml) als Feststoffe vorgelegt und mit 1.50 ml 1-Octen (9.60 mmol) versetzt. Anschließend wurde bei Raumtemperatur ein 1:1 Gasgemisch aus CO/H₂ bis zu einem Druck von 60 bar aufgepreßt. Nach ca. 20 Minuten wurden 13.6 g CO₂ mittels Kompressor eingefüllt. Anschließend wurde die Reaktionsmischung auf 60 °C aufgeheizt, wobei sich ein Gesamtdruck von 220 bar einstellte. Die gebildete überkritische Phase war homogen und gelb gefärbt. Der Innendruck sank innerhalb der Reaktionszeit von 19 h um etwa 25 bar auf 190 bar ab. Die ¹H-NMR-spektroskopische Analyse unmittelbar nach Erreichen des überkritischen Zustandes und nach 19 h ergab, daß im überkritischen Medium ein Umsatz von 92 % zu 1-Nonanal (n-Aldehyd) und 2-Nonanal (iso-Aldehyd) bei einem n/iso-Verhältnis von 4.6 erzielt worden war.

### Beispiel 10:

### Versuchte Hydroformylierung von 1-Octen in scCO₂ mit [(η⁴-C₈H₁₂)Rh(hfacac)]/TPP als Katalysator:

mg [(η⁴-C₈H₁₂)Rh(hfacac)] (0.05 mmol) und 72.5 mg (0.28 mmol) TPP wurden in einem mit Argon gespülten Druckreaktor (*V* = 20 ml) als Feststoffe vorgelegt und mit 1.50 ml (9.6 mmol) 1-Octen versetzt. Ein Teil der Feststoffe löste sich in dem Substrat unter Bildung einer rotbraunen Lösung. Anschließend wurden bei Raumtemperatur zuerst 60 bar eines 1:1 Gasgemisches aus CO/H₂ aufgepreßt und dann nach ca. 20 Minuten mit Hilfe eines Kompressors 13.6 g CO₂ eingefüllt. Nachdem die Reakionsmischung auf 60 °C aufgeheizt wurde, stellte sich ein Innendruck von 225 bar ein, der sich im weiteren Verlauf nicht mehr veränderte. Die gebildete überkritische Phase war farblos und es lag ein unlöslicher Feststoff vor. Die ¹H-NMR spektroskopische Analyse einer Probe, welche unmittelbar nach Erreichen des überkritischen Zustandes (visuelle Kontrolle) entnommen wurde, zeigte, daß ein Umsatz von 26 % zu 1-Nonanal (n-Aldehyd) und 2-Nonanal (iso-Aldehyd) bei einem n/iso-Verhältnis von 3.5 erreicht wurde. Nach 18 h im überkritischen Zustand wurde entspannt und die Produkte und Edukte isoliert. Erneute Analyse mittels ¹H-NMR ergab, daß im überkritischen Zustand kein weiterer Aldehyd mehr gebildet worden war.

### Beispiel 11:

Dieses Beispiel zeigt die Anwendbarkeit der Methode auf die Verwendung von Arylphosphiten:

### Darstellung von Tris-[4-(1H,1H,2H,2H-perfluoroctyl)phenyl]phosphit (XI):

Das Arylphosphit **XI** wurde in Anlehnung an Beispiel (1) und (3) nach Schema 3 gewonnen.

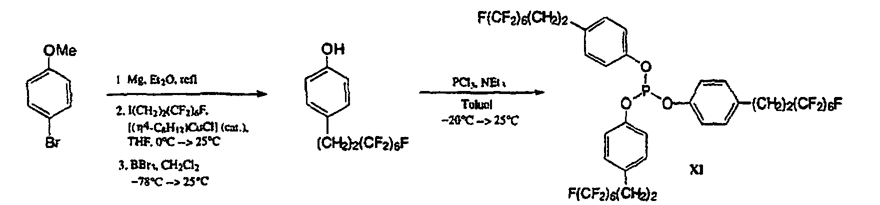

| | | | | |
|---|---|---|---|---|
| **GC-Massen- spektrum (EI)** | m/z (%) 1348(25) [M^{•+}] | | | |
| **Elementar- analyse** | ber. gef. | C: 37.41 C: 37.29 | H: 1.79 H: 1.88 | P: 2.30 P: 2.23 |
| ^{**31**}**P-NMR:** (CDd₃) | δ128.5 | | | |

### Schema 3: Synthese und ausgewählte analytische Daten des Arylphosphits XI.

Das Arylphosphit **XI** wurde in Anlehnung an Beispiel 9 in der Hydroformylierung von 1-Octen eingesetzt (Bedingungen: T=65°C, P(H₂/CO)=20 bar, 1-Octen:[Rh]:[P]=2175:1:10). NMR-Spektroskopische und gaschromatographische Analyse der Reaktionsmischung ergab 50% Umsatz nach 7.5 h und 98% Umsatz (n/iso = 5.6, ermittelt durch ¹H-NMR) nach 88 h.

### Beispiel 12:

Dieses Beispiel zeigt die Anwendbarkeit der Methode bei Verwendung der Reaktionsgemische in der Hydrierung polarer Doppelbindungen, speziell in der enantioselektiven Hdrierung von Iminen. Der Kontrollversuch belegt den positiven Einfluß der Perfluoralkyl-Reste.

Der perfluorierte chirale Ligand **(XIIb)** und die unsubstituierte Stammverbindung **(XIIa)** wurden nach bekannten Methoden (P. von Matt et al., *Tetrahedron: Asymmetrie* **1994,** *5,* 573) hergestellt, wobei zur Synthese von **XIIb** die Verbindung **III** anstelle von Ph₂PCl verwendet wurde. Die Iridiumkomplexe [(**XII**)Ir(cod)][BPh₄] wurden unter den in in Schema 4 zusammengefaßten Bedingungen für die enantioselektive Hydrierung von Ph(CH₃)C=NPh in scCO₂ eingesetzt, wobei **XIIb** sowohl hinsichtlich Aktivität als auch Selektivität der Stammverbindung **XIIa** überlegen ist.

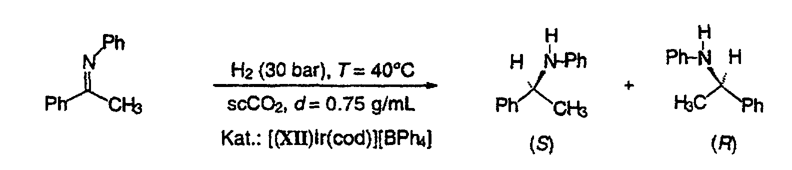

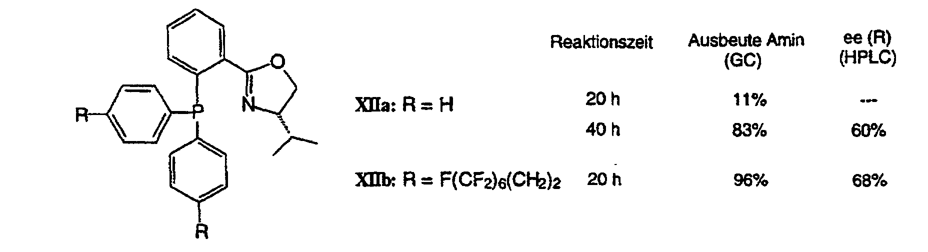

### Schema 4: Verwendung perfluorierter Liganden für die enantioselektive Hydrierung von Iminen in scCO₂.

### Beispiel 13:

Dieses Beispiel zeigt die Anwendbarkeit der Methode bei der Verwendung der Reaktionsmischungen in C-C-Kupplungsreaktionen, speziell in der Polymerisation von Phenylacetylen. Polyphenylacetylen ist aufgrund seiner nicht-linearen optischen und magnetischen Eigenschaften ein wichtiger polymerer Werkstoff in den Materialwissenschaften (G. Costa, in: *Comprehensive Polymer Science, Vol. 4*, G. Allen, J.C. Bevington (Hrsg.), Pergamon Press, Oxford, **1989,** S. 155ff).

7.0 mg (2.36 x 10⁻² mmol) [(η⁴-C₇H₈)Rh(acac)] (acac = acteylacetonat) werden zusammen mit 30 mg (2.31 x 10⁻² mmol) {4-F(CF₂)₆(CH₂)₂C₆H₄}₃P (Synthese analog **IV)** unter Ar-Atmosphäre (Glove-Box) in einen Edelstahl-Hochdruckreaktor mit Sichtfenster (V = 27 ml), PTFE-Rührkern, Bohrungen für Temperaturfühler, Absperr- und Kugelventil eingewogen und mit ca. 10 Äquivalenten NEt₃ versetzt. Die Dosiervorrichtung mit Gasvorratsbehälter wird über Swagelok-Verschraubung an dem Kugelventil des Autoklaven montiert und mit 0.25 ml (2.27 mmol) Phenylacetylen befüllt. Über das Nadelventil wird mit Hilfe eines Kompressors CO₂ (20.9 g, d = 0.78 g ml⁻¹, 83 bar) in den Reaktor gefüllt und die Reaktionsmischung unter Rühren auf 40 - 42 °C erhitzt, wobei der Druck auf 130 - 135 bar ansteigt und sich eine homogene gelbe Phase ausbildet. Das Gasvorratsbehältnis wird mit 150 - 180 bar Argon befüllt, die Dosiervorrichtung unter Druck gesetzt, durch Öffnen des Kugelventils zum Reaktorraum das Monomer eingeschossen und die Dosiervorrichtung mehrmals mit Argon-Druck gespült. Es ist ein spontaner Farbumschlag nach Orange zu beobachten und nach wenigen Sekunden scheidet sich bereits oranger Niederschlag am Sichtfenster des Autoklaven ab. Nach zwei Stunden Rühren wird der Reaktor auf 0 °C gekühlt und entspannt, anschließend unter Argon-Atmosphäre mit 20 ml THF und 1 ml Eisessig versetzt und über Nacht gerührt. Die rote Lösung wird zur Fällung des Polyphenylacetylens unter kräftigem Rühren in einen 2-Hals-Kolben mit 200 ml MeOH gehebert, feste Rückstände werden getrennt isoliert. Nach Filtration und Waschen mit MeOH und Aceton werden die Produktchargen im ÖV getrocknet.

| | |
|---|---|
| **Gesamtausbeute** | 213 mg (2.09 mmol, 92 %), orange Plättchen (*cis*-transoidal) + rote Kristalle (*cis*-cisoidal) im Gewichtsverhältnis 40: 60 |
| ^{**1**}**H-NMR, CDCl**_{**3**} **200.1 MHz** (lösliche Charge, *cis-* transoidales PPA) | δ 6.93 - 6.91 (m, 3H, *meta/para*), 6.65 - 6.61 (m, 2H, *ortho*), 5.82 (s, 1H, vinyl) ⇒ σ_{cis} ≈ 77 - 78 %. |

### Beispiel 14:

Dieses Experiment zeigt die Möglichkeit der Abtrennung von Produkt und Katalysator am Beispiel der Hydroformylierung von 1-Octen.

In einem 275 ml Fensterautoklaven wurden 14.4 mg (0.034 mmol) [(η⁴-C₈H₁₂)Rh(hfacac)], 337 mg (0.260 mmol) {4-F(CF₂)₆(CH₂)₂C₆H₄}₃P (Synthese analog **IV**) und 10.8 g (96 mmol) 1-Octen getrennt voneinander vorgelegt. Nach dem Verschließen des Reaktors wurden 20 bar Synthesegas zugegeben und mittels Kompressor soviel CO₂ aufgepreßt, daß sich bei Raumtemperatur ein Gesamtdruck von 120 bar einstellte. Dann wurde unter Rühren auf 65°C aufgeheizt. Unter Ausbildung einer homogen gelben Phase stellte sich ein Innendruck von ca. 210 bar ein. Nach 24 h wurde ein Innendruck von 190 bar abgelesen. Es wurde auf Raumtemperatur abgekühlt, wobei sich ein Zweiphasensystem bestehend aus einer farblosen, gasförmigen Phase und einer gelben, flüssigen Phase bildete.

Nach weiteren 24 h wurde durch vorsichtiges Entspannen über ein am Reaktorkopf angebrachtes Ventil CO₂ abgenommen, bis die Volumina der im Reaktor befindlichen flüssigen und der gasförmigen Phase etwa gleich waren. Anschließend wurde nach Schließen des Ventils auf 60°C aufgeheizt. Hierbei verringert sich das Volumen der flüssigen Phase und es stellte sich ein Innendruck von 100 bar ein.

Nun wurde über das obere Ventil eine konstante Menge (ca. 10 l/min) der oberen Phase entnommen und durch einen Abscheider geleitet. Um den Druck im Reaktor konstant zu halten, wurde über ein am Reaktorboden angebrachtes Ventil laufend CO₂ zugegeben. Nach ca. 15 Minuten hatten sich im Abscheider 4 ml einer farblosen Flüssigkeit angesammelt. ¹H-NMR-spektroskopische Analyse ergab, daß das Gemisch zu 95% aus 1-Nonanal und 2-Nonanal (n/iso = 2.5) bestand. Durch Atomabsorptionsspektroskopie (AAS) wurde ein Rh-Gehalt von 9 ppm in der abgeschiedenen Aldehyd-Mischung ermittelt.

## Patentansprüche

1. Reaktionsgemische bestehend im wesentlichen aus Reaktanden, einem Katalysator und Kohlendioxid im überkritischen Zustand (scCO₂), dadurch gekennzeichnet, daß der Katalysator oder Bestandteile davon Phosphorverbindungen sind, die Perfluoralkylsubstituenten der allgemeinen Formel R¹ [R¹= -(CH₂)ₓ(CF₂)_{y}E (E = F, H, x = 0 - 4, y = 2- 12)] enthalten.

2. Reaktionsgemische nach Anspruch 1, dadurch gekennzeichnet, daß die Phosphorverbindungen einen oder mehrere Arylreste enthalten, an welche ein oder mehrere Perfluoralkylketten der allgemeinen Formel R¹ gebunden sind.

3. Reaktionsgemische nach Anspruch 1 oder 2, dadurch gekennzeichnet, daß der Katalysator ein oder mehrere Übergangsmetallatome enthält.

4. Reaktionsgemische nach Anspruch 3, dadurch gekennzeichnet, daß als Übergangsmetall Rhodium oder Ruthenium oder Iridium eingesetzt wird.

5. Verfahren zur Durchführung katalytischer Reaktionen, dadurch gekennzeichnet, daß sie in einem Reaktionsgemisch nach Ansprüchen 1 bis 4 durchgeführt werden.

6. Verfahren nach Anspruch 5, dadurch gekennzeichnet, daß die katalytische Reaktion eine Hydrierung ist.

7. Verfahren nach Anspruch 6, dadurch gekennzeichnet, daß die Hydrierung eine chemoselektive Hydrierung von Polyenen zu Monoenen ist.

8. Verfahren nach Anspruch 7, wobei Isopren als Polyen eingesetzt wird.

9. Verfahren nach Anspruch 6, dadurch gekennzeichnet, daß die Reaktion die Hydrierung einer polaren Doppelbindung ist.

10. Verfahren nach Anspruch 9, dadurch gekennzeichnet, daß die polare Doppelbindung eine C=N-Doppelbindung ist.

11. Verfahren nach Anspruch 5, dadurch gekennzeichnet, daß die katalytische Reaktion eine Hydroformylierung von Olefinen oder von Mischungen von Olefinen ist.

12. Verfahren nach Anspruch 11, wobei Olefine der Kettenlänge C₄-C₂₀ eingesetzt werden.

13. Verfahren nach Anspruch 12, wobei 1-Octen als Olefin eingesetzt wird.

14. Verfahren nach Anspruch 5, dadurch gekennzeichnet daß die Reaktion eine C-C-Verknüpfungsreaktion ist.

15. Verfahren nach Anspruch 14, dadurch gekennzeichnet, daß die Reaktion eine Polymerisationsreaktion ist.

16. Verfahren nach Anspruch 15, dadurch gekennzeichnet, daß das Monomer der Polymerisation ein Acetylenderivat ist.

17. Verfahren nach Anspruch 5, dadurch gekennzeichnet, daß Produkt und Katalysator mit Hilfe der extraktiven Eigenschaften von scCO₂ voneinander getrennt werden, wobei der Katalysator in aktiver Form zurückgewonnen werden kann.

18. Verfahren nach Anspruch 17, dadurch gekennzeichnet, daß durch Variation von Druck und/oder Temperatur die Dichte des überkritischen Reaktionsmediums so eingestellt wird, daß die Extraktion des Produkts aus dem Reaktionsgemisch erreicht wird.

19. Verfahren nach Anspruch 17, dadurch gekennzeichnet, daß durch Variation von Druck und/oder Temperatur die Dichte des überkritischen Reaktionsmediums so eingestellt wird, daß die Extraktion des Katalysators von Produkt und Reaktionsgemisch erreicht wird.

## Claims

1. Reaction mixtures, essentially consisting of reactants, a catalyst and carbon dioxide in a supercritical state (scCO₂), characterized in that said catalyst or components thereof are phosphorus compounds containing perfluoroalkyl substituents of general formula R¹ [R¹= -(CH₂)ₓ(CF₂)_{y}E (E = F, H, x = 0 - 4, y = 2 - 12)].

2. The reaction mixtures according to claim 1, characterized in that said phosphorus compounds contain one or more aryl residues to which one or more perfluoroalkyl chains of general formula R¹ are attached.

3. The reaction mixtures according to claim 1 or 2, characterized in that said catalyst contains one or more transition metal atoms.

4. The reaction mixtures according to claim 3, characterized in that rhodium or ruthenium or iridium is employed as said transition metal.

5. A process of performing catalytic reactions, characterized in that the reactions are performed in a reaction mixture according to claims 1 to 4.

6. The process according to claim 5, characterized in that said catalytic reaction is a hydrogenation.

7. The process according to claim 6, characterized in that said hydrogenation is a chemoselective hydrogenation of polyenes to monoenes.

8. The process according to claim 7, wherein isoprene is used as said polyene.

9. The process according to claim 6, characterized in that said reaction is the hydrogenation of a polar double bond.

10. The process according to claim 9, characterized in that said polar double bond is a C=N double bond.

11. The process according to claim 5, characterized in that said catalytic reaction is a hydroformylation of olefins or mixtures of olefins.

12. The process according to claim 11, wherein olefins having a chain length of C₄-C₂₀ are used.

13. The process according to claim 12, wherein 1-octene is used as said olefin.

14. The process according to claim 5, characterized in that said reaction is a C-C bond-forming reaction.

15. The process according to claim 14, characterized in that said reaction is a polymerization reaction.

16. The process according to claim 15, characterized in that the monomer of said polymerization is an acetylene derivative.

17. The process according to claim 5, characterized in that the product and the catalyst are separated using the extractive properties of scCO₂, the catalyst being recovered in an active form.

18. The process according to claim 17, characterized in that the density of the supercritical reaction medium is adjusted by a variation of the pressure and/or temperature to achieve extraction of the product from the reaction mixture.

19. The process according to claim 17, characterized in that the density of the supercritical reaction medium is adjusted by a variation of the pressure and/or temperature to achieve extraction of the catalyst from the product and reaction mixture.

## Revendications

1. Mélanges réactionnels constitués pour l'essentiel de réactifs, d'un catalyseur et de dioxyde de carbone à l'état surcritique (scCO₂), caractérisés en ce que le catalyseur ou des constituants de ce dernier sont des composés du phosphore qui contiennent des substituants perfluoralkyle de formule générale R¹[R¹=-(CH₂)ₓ(CF₂)_{y}E (E = F, H, x = 0-4, y = 2-12)].

2. Mélanges réactionnels selon la revendication 1, caractérisés en ce que les composés du phosphore contiennent un ou plusieurs radicaux aryle auxquels sont liées une ou plusieurs chaînes perfluoralkyle de formule générale R¹.

3. Mélanges réactionnels selon la revendication 1 ou 2, caractérisés en ce que le catalyseur contient un ou plusieurs atomes de métaux de transition.

4. Mélanges réactionnels selon la revendication 3, caractérisés en ce qu'on utilise en tant que métal de transition du rhodium ou du ruthénium ou de l'iridium.

5. Procédé pour mettre en oeuvre des réactions catalytiques, caractérisé en ce qu'elles sont mises en oeuvre dans un mélange réactionnel selon les revendications 1 à 4.

6. Procédé selon la revendication 5, caractérisé en ce que la réaction catalytique est une hydrogénation.

7. Procédé selon la revendication 6, caractérisé en ce que l'hydrogénation est une hydrogénation chimiosélective de polyènes en monoènes.

8. Procédé selon la revendication 7, dans lequel on utilise de l'isoprène en tant que polyène.

9. Procédé selon la revendication 6, caractérisé en ce que la réaction est l'hydrogénation d'une double liaison polaire.

10. Procédé selon la revendication 9, caractérisé en ce que la double liaison polaire est une double liaison C=N.

11. Procédé selon la revendication 5, caractérisé en, ce que la reaction catalytique est une hydroformylation d'oléfines ou de mélanges d'oléfines.

12. Procédé selon la revendication 11, dans lequel on utilise des oléfines ayant une chaîne en C₄-C₂₀.

13. Procédé selon la revendication 12, dans lequel on utilise du 1-octène en tant qu'oléfine.

14. Procédé selon la revendication 5, caractérisé en ce que la réaction est une réaction de liaison C-C.

15. Procédé selon la revendication 14, caractérisé en ce que la réaction est une réaction de polymérisation.

16. Procédé selon la revendication 15, caractérisé en ce que le monomère de la polymérisation est un dérivé de l'acétylène.

17. Procédé selon la revendication 5, caractérisé en ce que le produit et le catalyseur sont séparés l'un de l'autre à l'aide des propriétés extractives du scCO₂, le catalyseur pouvant être récupéré sous une forme active.

18. Procédé selon la revendication 17, caractérisé en ce que, par variation de la pression et/ou de la température, on ajuste la masse volumique du milieu réactionnel surcritique de façon à obtenir l'extraction du produit à partir du mélange réactionnel.

19. Procédé selon la revendication 17, caractérisé en ce que, par variation de la pression et/ou de la température, on ajuste la masse volumique du milieu réactionnel surcritique de façon à obtenir l'extraction du catalyseur à partir du produit et du mélange réactionnel.
